# EUROPÄISCHE PATENTSCHRIFT

(11) **EP 2 229 930 B1**
(45) Veröffentlichungstag und Bekanntmachung des Hinweises auf die Patenterteilung: **12.12.2012**
(21) Anmeldenummer: 09155324.8
(22) Anmeldetag: 17.03.2009
(51) Int. Cl.: A61K 6/00

(54) **Dentale universelle Haftvermittlerzusammensetzung**
Dental universal adhesive compound
Composé dentaire adhésif universel

(43) Veröffentlichungstag der Anmeldung: 22.09.2010
(73) Patentinhaber: Ivoclar Vivadent AG, 9494 Schaan (LI)
(72) Erfinder: Salz, Ulrich Dr., 88131, Lindau (DE); Moszner, Norbert Prof. Dr., 9493 Mauren (LI); Zeuner, Frank Dr., 9488, Schellenberg (LI); Rheinberger, Volker Dr., 9490, Vaduz (LI); Bock, Thorsten Dr., 6806, Tosters (AT)
(74) Vertreter: UEXKÜLL & STOLBERG

(56) Entgegenhaltungen:
- EP-A- 1 911 434
- EP-A- 2 036 532
- DE-A1- 19 503 551
- DE-A1-102005 002 750
- KEIICHI YOSHIDA; KOHJI KAMADA; MITSURU ATSUTA: "Shear Bond Strength of a New Resin Bonding System to Different Ceramic Restorations" INT J PROSTHODONT, Bd. 20, Nr. 4, 2007, Seiten 417-418, XP002529925

## Beschreibung

Die vorliegende Erfindung betrifft eine dentale Haftvermittlerzusammensetzung zur adhäsiven Verbindung von unterschiedlichen metallischen oder keramischen Dentalmaterialien mit radikalisch härtenden Dentalmaterialien, bevorzugt Kompositen.

Bei der Zahnrestauration ist es häufig notwendig, radikalisch härtende Dentalmaterialien, insbesondere Formulierungen, die anorganische Füllstoffe in einer radikalisch härtbaren organischen Matrix aufweisen (sogenannte Komposite), mit anderen dentalen Restaurationsmaterialien auf metallischer oder mineralischer Basis zu kombinieren. Im Einzelnen unterscheidet man bei den Restaurationsmaterialien zwischen folgenden Materialtypen: Edelmetalle (z.B. Gold, Platin, Palladium, Silber und deren Legierungen), Nichtedelmetalle (z.B. Chrom, Nickel, Molybdän, Titan und deren Legierungen), silicatische Keramiken (z.B. Feldspat, Quarz, auf Leucit basierende Keramiken bzw. Glaskeramik) oder nichtsilicatische Keramiken (z.B. yttriumstabilisiertes Zirkonoxid, Aluminiumoxid, glasinfiltriertes Aluminiumoxid). Um die Haftung zwischen den vorgenannten Restaurationsmaterialien und dem radikalisch härtenden Dentalmaterial zu verbessern, werden Haftvermittler (Primer) eingesetzt. Bislang bekannte Primer zur adhäsiven Befestigung von dentalen Restaurationsmaterialien eignen sich in der Regel jeweils nur für einen der genannten Materialtypen und vermitteln keine klinisch brauchbare Haftung auf anderen Substraten.

In DE 10 2005 002 750 A1 ist ein Primer für dentale Edelmetalllegierungen offenbart, der spezielle mit polymerisierbaren Gruppen substituierte Disulfide enthält.

EP 0 224 319 A1 beschreibt eine Primer-Zusammensetzung zur Haftverbesserung auf verschiedenen keramischen Materialien, die ein zu einem organofunktionellen Silanol hydrolysierbares Silan enthält. Auch wenn in EP 0 224 319 A1 eine Haftverbesserung auch auf metallischen Materialien ausgewiesen ist, so zeigt sich in der Praxis, dass die beschriebenen Primer nur auf silicatischen Keramiken Wirkung haben.

Gegenstand von JP 2601254 B2 ist ein dentaler Primer für Keramiken und Metall, der die Kombination eines organofunktionellen Silans mit speziellen (meth)acryloyloxyfunktionellen Phosphorsäuremonoestern enthält. JP 2593850 B2 beschreibt eine adhäsive Dentalzusammensetzung, die u.a. ein organofunktionelles Silan und eine saure organische Phosphorverbindung mit einer radikalisch polymerisierbaren Doppelbindung enthält. Die Zusammensetzung soll die Bindung sowohl an Metalle als auch Keramik ermöglichen. Auf Edelmetalloberflächen, wie etwa Gold, wird jedoch mit den beiden vorgenannten Zusammensetzungen keine chemische Anbindung bewirkt und daher kann mit diesen Zusammensetzungen kein dauerhafter Verbund zwischen Metall und einem radikalisch härtenden Dentalmaterial entstehen, insbesondere wegen der unterschiedlichen Wärmeausdehnungskoeffizienten der zu verbindenden Materialien.

Auch die WO 2008/053990 offenbart eine adhäsive Dentalzusammensetzung, die die Kombination eines Silan-Kupplungsmittels mit speziellen (meth)acryloyloxyfunktionellen Phosphorsäuremonoestern enthält. Die Zusammensetzung soll eine gute Haftung sowohl an Dentalkeramiken als auch an organische Kompositen zeigen, die anorganische Materialen enthalten.

In der wissenschaftlichen Literatur sind Angaben zu finden, dass sich spezifische Haftmonomere besonders für spezielle Metall-, beziehungsweise Keramiktypen eignen (siehe zum Beispiel Kern M, Wegner MS, "Bonding to Zirconia Ceramic: Adhesion Methods and Their Durability", Dent Mater 1998, 14; 64-71; Yoshida K, Kamada K, Atsuta M, "Shear Bond Strength of a New Resin Bonding System to Different Ceramic Restorations", Int, J. Prosthodont 2007, 20; 417-418).

Nachteilig bei der Anwendung von substratspezifischen Primersystemen ist jedoch, dass durch die Vielfalt der Substrattypen eine entsprechende Anzahl an Primern erforderlich wird. Mit der Anzahl an substratspezifischen Primersystemen steigt bei klinischer Anwendung aber auch die Verwechslungsgefahr und somit auch das Risiko des klinischen Misserfolgs. Zudem ist eine exakte Applikation eines substratspezifischen Primers klinisch in vielen Fällen nicht möglich, wie zum Beispiel bei einer Reparatur einer frakturierten Keramikverblendung, da mehrere Substrate auf engem Raum in direkter Nachbarschaft vorliegen.

Der Erfindung liegt demgemäß die Aufgabe zugrunde, einen universellen Haftvermittler bereitzustellen, der sich durch eine gute Verbundwirkung zwischen einem radikalisch härtenden Dentalmaterial, insbesondere einem Komposit, und einer Vielzahl von anderen dentalen Restaurationsmaterialien auszeichnet. Es soll auch nach Thermowechselbelastung eine gute Verbundwirkung bestehen bleiben.

Die Aufgabe wird gelöst durch eine Haftvermittlerzusammensetzung, enthaltend
(i) mindestens ein Alkoxysilan-Monomer der allgemeinen Formel

   R¹nSi(OR²)₄₋ₙ (I),

   worin
   - R¹: für einen Rest steht, der mindestens eine polymerisierbare Gruppe aufweist,
   - R²: für einen C₁- bis C₈-Alkylrest, vorzugsweise C₁- bis C₄-Alkylrest steht und
   - n: 1,2 oder 3 ist,
   wobei die Reste R¹ und R² jeweils gleich oder unterschiedlich sein können;
(ii) mindestens ein Phosphorsäureester-Monomer der allgemeinen Formel

   O=P(OR³)ₘ(OR⁴)₃₋ₘ (II),

   worin
   - R³: für einen Rest steht, der mindestens eine polymerisierbare Gruppe aufweist,
   - R⁴: für einen Rest ausgewählt aus H, Silyl, bevorzugt Si-Me₃, und C₁- bis C₁₆-Alkyl, insbesondere C₁- bis C₄-Alkyl, steht und
   - m: 1 oder 2 ist,
   wobei die Reste R³ und R⁴ jeweils gleich oder unterschiedlich sein können;
(iii) mindestens ein schwefelhaltiges Monomer der allgemeinen Formel

   R⁵-R⁶-Sₓ-R⁶-R⁵ (IIIa)

   oder

   R⁵-R⁶-Sₓ-R⁷ (IIIb),

   worin
   - x: eine ganze Zahl von 1 bis 8, vorzugsweise 2, ist,
   - R⁵: für einen Rest steht, der mindestens eine polymerisierbare Gruppe aufweist, und die beiden R⁵ in Formel (IIIa) gleich oder unterschiedlich sein können,
   - R⁶: für einen unsubstituierten oder substituierten C₁- bis C₃₀-Alkylenrest steht, und die beiden R⁶ in Formel (IIIa) gleich oder unterschiedlich sein können,
   - R⁷: für H oder einen unsubstituierten oder substituierten C₁- bis C₂₆-Alkylrest steht,
   wobei die beiden Reste R⁶ in Formel (IIIa) bzw. R⁶ und R⁷ in Formel (IIIb) miteinander verbunden sein können, um mit Sₓ einen Heterocyclus zu bilden, und
(iv) organisches Lösungsmittel.

Geeignete Substituenten der obengenannten Alkylen- bzw. Alkylreste (R⁶, R⁷) sind Aryl-, Alkylaryl-, Heteroalkyl-, Heteroaryl-, Heteroalkylaryl-, Urethan-, Halogen, Isocyanat-, Ureid-, und/oder- Imidazolinylgruppen ebenso wie Aryl-, Alkylaryl-, Heteroalkyl-, Heteroaryl-, und/oder Heteroalkylarylreste, die mit Urethan-, Halogen, Isocyanat-, Ureid-, Imidazolinylgruppen, Acryloyloxy- und / oder Methacryloyloxy-Gruppen, insbesondere mit Urethan-, Halogen, Isocyanat-, Ureid-, und/oder-Imidazolinylgruppen substituiert sind.

Die Erfindung betrifft auch die Verwendung der Haftvermittlerzusammensetzung in der Zahnheilkunde und Zahntechnik, insbesondere die Verwendung zur adhäsiven Verbindung von metallischen oder keramischen Dentalmaterialien mit radikalisch härtenden Dentalmaterialien, bevorzugt Kompositen und auf Kompositen basierenden Befestigungsmaterialien (Zementen).

Komponente (i), das mindestens eine Alkoxysilan-Monomer der Formel R¹ₙSi(OR²)₄₋ₙ (I), weist neben der hydrolysierbaren Alkoxygruppe -OR² mindestens einen Rest R¹ auf, der mindestens eine, bevorzugt genau eine polymerisierbare Gruppe enthält. Typischerweise handelt es sich um eine radikalisch polymerisierbare Gruppe. Bevorzugt hat das Alkoxysilan ein oder zwei R¹-Reste. Der R¹-Rest ist vorzugsweise nicht hydrolysierbar. Bevorzugt enthält R¹ eine ethylenisch ungesättigte Doppelbindung. Beispielsweise kann R¹ eine (Meth)acryloyloxygruppe (H₂C=C(R⁸)-CO-O- mit R⁸ = CH₃ oder H), eine (Meth) acryloylaminogruppe (H₂C=C(R⁹)-CO-NH- mit R⁹ = CH₃ oder H), eine Vinyl-, Allyl- oder Styrylgruppe enthalten, wobei die genannten Gruppen unsubstituiert oder durch geeignete Substituenten substituiert sein können. Bevorzugte Reste R¹ umfassen (Meth)acryloyloxyalkyl, bevorzugt (Meth)acryloyloxy-C₂-C₁₆-alkyl, besonders bevorzugt (Meth)acryloyloxypropyl; (Meth)acryloylaminoalkyl, bevorzugt (Meth)acryloylamino-C₂-C₁₆-alkyl, besonders bevorzugt (Meth)acryloylaminopropyl; Vinyl; Allyl und Styryl.

Der Alkylrest R² der Alkoxygruppe in Formel (I) weist 1 bis 8 C-Atome auf und ist vorzugsweise geradkettig oder verzweigt. Bevorzugt ist R² ein Methyl-, Ethyl-, n- oder i-C₃-C₈-Rest, besonders bevorzugt Methyl oder Ethyl.

Für die vorliegende Erfindung besonders geeignete Alkoxysilan-Monomere (i) sind:
3-Methacryloyloxypropyltrimethoxysilan (= 3-Trimethoxysilylpropyl-methacrylate) (MPTMS) 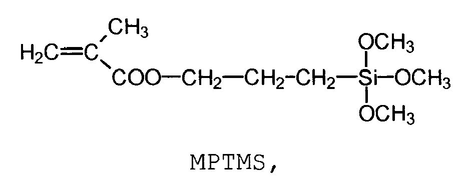
3-Methacryloyloxypropyltriethoxysilan (= 3-Triethoxysilylpropyl-methacrylate) (MPTES) 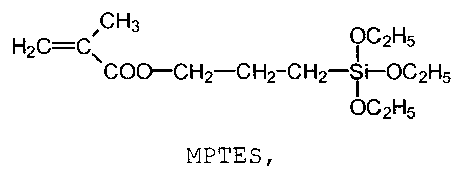
Di(3-methacryloyloxypropyl)dimethoxysilan (DPDMS) 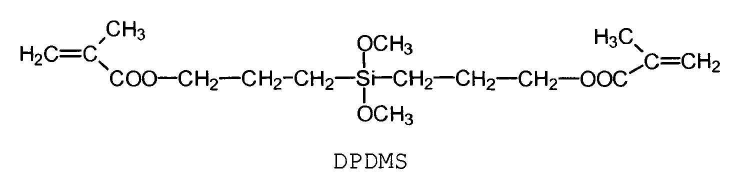
und Methacrylsäure-(3-trimethoxysilylpropyl)amid (MTPA)

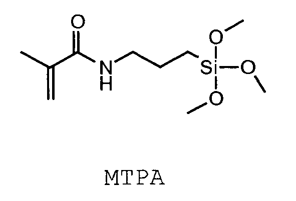

Das am meisten bevorzugte Alkoxysilan-Monomer ist 3-Methacryloyloxypropyltrimethoxysilan.

Komponente (i) ist typischerweise in der erfindungsgemäßen Haftvermittlerzusammensetzung in einer Menge von 0,05 bis 25,0 Gew.-%, bevorzugt 0,2 bis 10,0 Gew.-% und besonders bevorzugt 0,5 bis 5,0 Gew.-% vorhanden, jeweils bezogen auf das Gesamtgewicht der Zusammensetzung.

Komponente (ii), das mindestens eine Phosphorsäureester-Monomer der allgemeinen Formel O=P(OR³)ₘ(OR⁴)₃₋ₘ (II), weist mindestens einen Rest R³ auf, der mindestens eine, bevorzugt eine oder zwei polymerisierbare Gruppen enthält. Typischerweise handelt es sich um (eine) radikalisch polymerisierbare Gruppe(n). Vorzugsweise hat der Phosphorsäureester genau einen R³-Rest und weist somit die folgende Formel (IIa) auf.

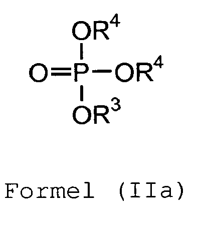

Bevorzugt enthält R³ in Formel (II) bzw. (IIa) mindestens eine ethylenisch ungesättigte Doppelbindung. Beispielsweise kann R³ mindestens eine (Meth)acryloyloxygruppe, eine (Meth)acryloylaminogruppe, eine Vinyl-, Allyl- oder Styrylgruppe oder eine Kombination derselben enthalten, wobei die genannten Gruppen unsubstituiert oder durch geeignete Substituenten substituiert sein können. Bevorzugte Reste R³ umfassen (Meth)acryloyloxyalkyl, bevorzugt (Meth)acryloyloxy-C₂-C₁₆-alkyl, besonders bevorzugt (Meth)acryloyloxy-C₄-C₁₄-alkyl, ganz besonders bevorzugt (Meth)acryloyloxy-C₆-C₁₀-alkyl; Di (meth) acryloyloxyalkyl, bevorzugt Di(meth)acryloyloxy-C₂-C₁₆-alkyl, besonders bevorzugt Di(meth)acryloyloxy-C₂-C₁₀-alkyl, besonders bevorzugt Di(meth)acryloyloxyisopropyl; (Meth)acryloylaminoalkyl, bevorzugt (Meth)acryloylamino-C₂-C₁₆-alkyl, besonders bevorzugt (Meth)acryloylamino-C₄-C₁₄-alkyl, ganz besonders bevorzugt (Meth)acryloylamino-C₆-C₁₀-alkyl; Vinyl; Allyl und Styryl.

Der R⁴-Rest ist ausgewählt aus H, Silyl, bevorzugt SiMe₃, und C₁- bis C₁₆-Alkyl, wobei der Alkylrest vorzugsweise geradkettig oder verzweigt, besonders bevorzugt Methyl, Ethyl oder ein n- oder i-C₃-C₁₆-Rest ist. In einer besonders bevorzugten Ausführungsform ist R⁴ gleich H, wobei die Dihydrogenphosphate (Phosphorsäuremonoester) die am meisten bevorzugten Phosphorsäureester-Monomere darstellen.

Für die vorliegende Erfindung besonders geeignete Phosphorsäureester-Monomere (ii) sind:
1-Methacryloyloxydecan-10-phosphat (MDP) 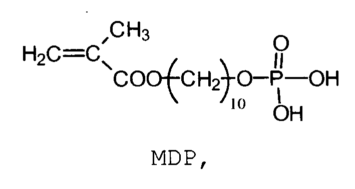
1-Methacryloyloxyhexan-6-phosphat (MHP) 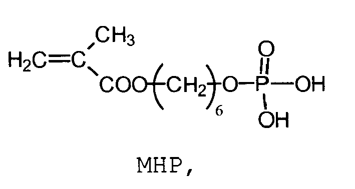
1-Methacryloylaminodecan-10-phosphat (MADP) 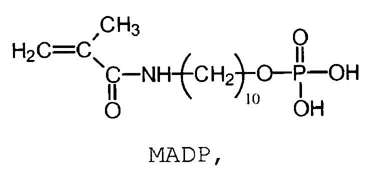
1-Acryloylaminohexan-6-phosphat (AAHP) 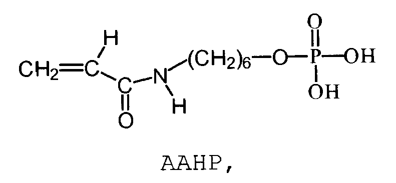
1,3-Dimethacryloyloxypropan-2-phosphat (DMPP) 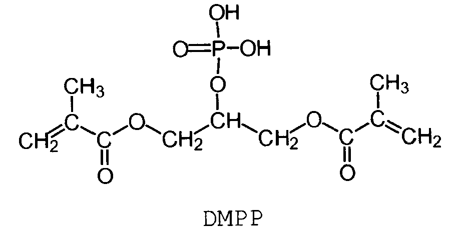
und 1,3-Dimethacryloylaminopropan-2-phosphat (DMAPP)

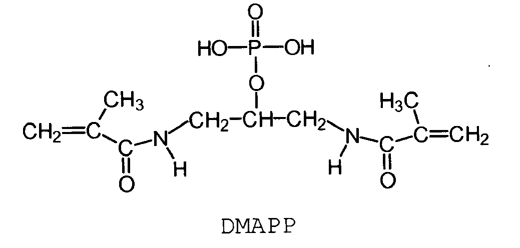

Die am meisten bevorzugten Phosphorsäureester-Monomere sind 1-Methacryloyloxydecan-10-phosphat und 1-Methacryloylaminodecan-10-phosphat.

Komponente (ii) ist typischerweise in der erfindungsgemäßen Haftvermittlerzusammensetzung in einer Menge von 0,05 bis 25,0 Gew.-%, bevorzugt 0,2 bis 10,0 Gew.-% und besonders bevorzugt 0,5 bis 5,0 Gew.-% vorhanden, jeweils bezogen auf das Gesamtgewicht der Zusammensetzung.

Auch Komponente (iii), das mindestens eine schwefelhaltige Monomer der allgemeinen Formel R⁵-R⁶-Sₓ-R⁶-R⁵ (IIIa) oder R⁵-R⁶-Sₓ-R⁷ (IIIb), weist mindestens einen Rest R⁵ auf, der mindestens eine, bevorzugt genau eine polymerisierbare Gruppe enthält. Vorzugsweise ist diese polymerisierbare Gruppe radikalisch polymerisierbar, daher weist R⁵ typischerweise eine ethylenisch ungesättigte Doppelbindung auf. Beispielsweise kann R⁵ eine (Meth)acryloyloxygruppe, eine (Meth)acryloylaminogruppe, eine Vinyl-, Allyl- oder Styrylgruppe enthalten, wobei die genannten Gruppen unsubstituiert oder durch geeignete Substituenten substituiert sein können. Bevorzugte Reste R⁵ umfassen (Meth)acryloyloxy, (Meth)acryloylamino, eine unsubstituierte oder substituierte Vinylgruppe, eine unsubstituierte oder substituierte Allylgruppe, eine unsubstituierte oder substituierte Styrylgruppe, eine unsubstituierte oder substituierte Vinylethergruppe, eine unsubstituierte oder substituierte Vinylestergruppe, eine unsubstituierte oder substituierte Allylethergruppe, eine unsubstituierte oder substituierte Allylestergruppe und Vinylcyclopropyl. Mögliche Substituenten sind z.B. -COOR⁸, worin R⁸ eine geradkettige oder verzweigte C₁- bis C₁₆-Alkylgruppe, vorzugsweise C₁- bis C₆-Alkylgruppe ist. In einer Ausführungsform ist R⁵ eine mit -COOR⁸, bevorzugt -COOEt, substituierte Allylestergruppe.

R⁶ steht für einen unsubstituierten oder substituierten C₂- bis C₃₀-Alkylenrest, wobei die beiden R⁶ in Formel (IIIa) gleich oder unterschiedlich sein können. Der C₂- bis C₃₀-Alkylenrest ist typischerweise geradkettig oder verzweigt. Vorzugsweise steht R⁶ für einen C₂- bis C₁₅-Alkylenrest, besonders bevorzugt für einen C₂- bis C₈-Alkylenrest.

R⁷ in Formel (IIIb) steht für H oder einen unsubstituierten oder substituierten C₁- bis C₂₆-Alkylrest. Der C₁- bis C₂₆-Alkylrest ist typischerweise geradkettig oder verzweigt. Vorzugsweise steht R⁷ für einen C₂- bis C₁₅-Alkylrest, besonders bevorzugt für einen C₂- bis C₈-Alkylrest.

Die gegebenenfalls vorhandenen Substituenten der Reste R⁶ und R⁷ sind wie oben definiert.

Bevorzugte schwefelhaltige Monomere sind die Disulfide, d.h. x = 2 in Formel (IIIa) oder (IIIb).

In einer Ausführungsform der vorliegenden Erfindung sind die beiden R⁶-Rest in Formel (IIIa) bzw. R⁶ und R⁷ in Formel (IIIb) miteinander verbunden, um mit Sₓ einen Heterocyclus zu bilden. Vorzugsweise enthält das schwefelhaltige Monomer der Komponente (iii) dann die folgende Struktureinheit 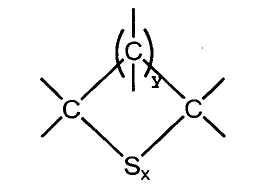 worin y = 1 bis 8 und die C-Atome mit H bzw. wie zuvor beschrieben substituiert sind. Besonders bevorzugt ist x = 2 und y = 1, d.h. das schwefelhaltige Monomer enthält einen Dithiolanring der folgenden Struktur:

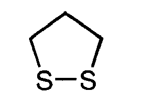

In einer Ausführungsform ist in Formel (IIIb) R⁶ = C₅ und R⁷ = C₂, die miteinander verbunden sind, um einen C₄-substituierten Dithiolanring zu bilden. In einer anderen Ausführungsform ist in (IIIa) R⁶ ein Ethylenrest.

Für die vorliegende Erfindung besonders geeignete schwefelhaltige Monomere sind:
Liponsäure-2-ethoxycarbonylallylester (LSEAE) 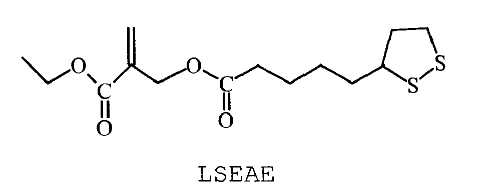
und 2,2-Bisacryloylamino-diethyldisulfid (BAADS).

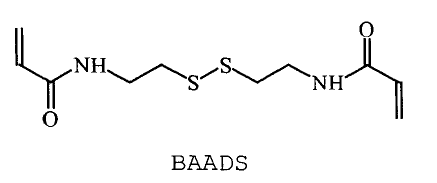

Komponente (iii) ist typischerweise in der erfindungsgemäßen Haftvermittlerzusammensetzung in einer Menge von 0,05 bis 25,0 Gew.-%, bevorzugt 0,2 bis 10,0 Gew.-% und besonders bevorzugt 0,5 bis 5,0 Gew.-% vorhanden, jeweils bezogen auf das Gesamtgewicht der Zusammensetzung.

Die vorliegende Haftvermittlerzusammensetzung kann als Komponenten (i), (ii) und (iii) auch Mischungen aus verschiedenen Monomeren des jeweiligen Typs enthalten.

Komponente (iv) der erfindungsgemäßen Haftvermittlerzusammensetzung ist ein organisches Lösungsmittel, typischerweise ein physiologisch verträgliches Lösungsmittel. Geeignete Lösungsmittel sind beispielsweise Alkohole, Ketone und Ester und deren Mischungen, wobei Methanol, Ethanol, Isopropanol, t-Butanol, Ethylacetat, Aceton, Methylethylketon und deren Mischungen bevorzugt sind. Besonders bevorzugt ist Ethanol. Typischerweise enthält die Haftvermittlerzusammensetzung 25 bis 98,5 Gew.-%, bevorzugt 35 bis 97 Gew.-% und besonders bevorzugt 45 bis 96 Gew.-% organisches Lösungsmittel (iv), jeweils bezogen auf das Gesamtgewicht der Zusammensetzung.

Weiterhin kann der erfindungsgemäße Haftvermittlerzusammensetzung zusätzliche Additive, etwa zur Verbesserung der mechanischen Eigenschaften oder zur Einstellung der Viskosität, enthalten. Beispielsweise kann die Haftvermittlerzusammensetzung einen Füllstoff (v), vorzugsweise einen anorganischen partikulären Füllstoffe enthalten. Die Füllstoffe weisen vorzugsweise eine Partikelgröße (bestimmt durch statische Lichtstreuung bzw. Laserbeugung) im Bereich von 10 bis 250 nm auf, wobei sphärische Partikel besonders bevorzugt sind. Bevorzugt sind anorganische Füllstoffe auf der Basis von Oxiden, insbesondere aus pyrogenen oder Fällungsprozessen. Geeignete Materialen sind zum Beispiel, aber nicht beschränkt auf ZrO₂, TiO₂, SiO₂, Al₂O₃, Ta₂O₅ und Yb₂O₃ ebenso wie Mischoxide aus mindestens zwei von SiO₂, ZrO₂, Ta₂O₅ und TiO₂. Besonders geeignet sind Füllstoffe, die mit polymerisationsfähigen Gruppen oberflächenmodifiziert sind. Falls vorhanden, ist der Füllstoff (v) in der Haftvermittlerzusammensetzung vorzugsweise in einer Maximalmenge von 10 Gew.-%, besonders bevorzugt 0,5 bis 5,0 Gew.-%, enthalten, jeweils bezogen auf das Gesamtgewicht der Zusammensetzung.

Vorzugsweise enthält die Haftvermittlerzusammensetzung gemäß der vorliegenden Erfindung einen Polymerisationsinitiator (vi).

Der Polymerisationsinitiator ist ein Initiator für die radikalische Polymerisation und wird ausgewählt, je nachdem, ob Strahlungshärtung (photochemische radikalische Polymerisation), Heißhärtung oder Härtung bei Raumtemperatur erwünscht ist. Unter dem Begriff "Initiator" sind auch Initiatorsysteme aus verschiedenen Verbindungen zu verstehen.

Beispiele für geeignete Photoinitiatoren umfassen Benzophenon, Benzoin sowie deren Derivate und α-Diketone und deren Derivate, wie 9,10-Phenanthrenchinon, 1-Phenyl-propan-1,2-dion, Diacetyl und 4,4'-Dichlorbenzil. Bevorzugt werden Campherchinon, 2,2-Methoxy-2-phenyl-acetophenon oder α-Diketone jeweils in Kombination mit Aminen als Reduktionsmittel, wie z.B. 4-(Dimethylamino)-benzoesäureester, N,N-Dimethylaminoethylmethacrylat, N,N-Dimethyl-sym.-xylidin oder Triethanolamin, sowie Acyl- oder Bisacylphosphinoxide, Monobenzoyl- oder Dibenzoylgermanium-Derivate verwendet.

Als Initiatoren für die Heißhärtung eignen sich beispielsweise Benzpinakol und 2,2'-Dialkylbenzpinakole.

Als Initiatoren für eine bei Raumtemperatur durchgeführte Polymerisation (Kalthärtung) können beispielsweise Redox-Initiatorkombinationen, wie z.B. Kombinationen von Benzoylperoxid mit N,N-Dimethyl-sym.-xylidin oder N,N-Dimethyl-p-toluidin, verwendet werden. Darüber hinaus sind auch Redoxsysteme, bestehend aus Peroxiden und Reduktionsmitteln, wie z.B. Ascorbinsäure, Barbituraten oder Sulfinsäuren, geeignet.

Der Polymerisationsinitiator (vi) wird vorzugsweise in einer Menge von 0,001 bis 3 Gew.-%, besonders bevorzugt 0,1 bis 0,2 Gew.-% bezogen auf das Gesamtgewicht der Zusammensetzung eingesetzt.

Außerdem kann die erfindungsgemäße Haftvermittlerzusammmensetzung Stabilisatoren (vii) und Polymerisationsinhibitoren (viii), wie etwa Methylhydrochinon, Butylhydroxytoluol (BHT) und Phenothiazin, enthalten.

Gemäß einer bevorzugten Ausführungsform enthält die Haftvermittlerzusammensetzung gemäß der vorliegenden Erfindung:
(a) 0,05 bis 25,0 Gew.-%, bevorzugt 0,2 bis 10,0 Gew.-% und besonders bevorzugt 0,5 bis 5,0 Gew.-% Alkoxysilan-Monomer (e) (i);
(b) 0,05 bis 25,0 Gew.-%, bevorzugt 0,2 bis 10,0 Gew.-% und besonders bevorzugt 0,5 bis 5,0 Gew.-% Phosphorsäureester-Monomer(e) (ii);
(c) 0,05 bis 25,0 Gew.-%, bevorzugt 0,2 bis 10,0 Gew.-% und besonders bevorzugt 0,5 bis 5,0 Gew.-% schwefelhaltige(s) Monomer(e) (iii);
(d) 25 bis 98,5 Gew.-%, bevorzugt 35 bis 97 Gew.-% und besonders bevorzugt 45 bis 96 Gew.-% organisches Lösungsmittel (iv);
(e) 0 bis 10 Gew.-% Füllstoff (v),
(f) 0,001 bis 3 Gew.-%, bevorzugt 0,1 bis 0,2 Gew.-% Polymerisationsinitiator (vi);
jeweils bezogen auf das Gesamtgewicht der Zusammensetzung.

Besonders bevorzugte erfindungsgemäße Haftvermittlerzusammensetzungen enthalten:
(A) eine Kombination von (i) 3-Methacryloyloxypropyltrimethoxysilan, (ii) 1-Methacryloyloxydecan-10-phosphat und (iii) Liponsäure-2-ethoxycarbonylallylester;
(B) eine Kombination von (i) 1-Methacryloylaminodecan-10-phosphat, (ii) Methacrylsäure-(3-trimethoxysilylpropyl)amid und (iii) Liponsäure-2-ethoxycarbonylallylester oder
(C) eine Kombination von (i) 1-Methacryloyloxydecan-10-phosphat, (ii) 3-Methacryloyloxypropyltrimethoxysilan und (iii) 2,2-Bisacryloylamino-diethyldisulfid.

Vorzugsweise werden diese Komponenten in den zuvor genanten Mengen und wahlweise mit den genanten optionalen Additiven eingesetzt.

Die erfindungsgemäße Haftvermittlerzusammensetzung findet vor allem in der Zahnheilkunde und Zahntechnik Verwendung und zeigt eine gute Verbundwirkung zwischen einem radikalisch härtenden Dentalmaterial und anderen dentalen Restaurationsmaterialien verschiedenen Typs. Radikalisch härtende Dentalmaterialien zur Verwendung mit der erfindungsgemäßen Haftvermittlerzusammensetzung sind bevorzugt dentale Füllungskomposite bzw. auf Kompositen basierende Befestigungsmaterialien (Zemente), aber auch Adhäsive und andere radikalisch härtende Dentalmaterialien. Dentale Restaurationsmaterialien auf denen die Haftvermittlerzusammensetzung gemäß der vorliegenden Erfindung angewandt werden kann und dann zu einem guten Verbund - auch nach Thermowechselbelastung - mit dem radikalisch härtenden Dentalmaterial führt, sind beispielsweise Edelmetalle, wie etwa Gold, Platin, Palladium, Silber und deren Legierungen; Nichtedelmetalle, wie etwa Chrom, Nickel, Molybdän, Titan und deren Legierungen; silicatische Keramiken, wie etwa Keramiken auf Basis von Feldspat und/oder Quarz, leucithaltige Keramiken und Glaskeramiken, z.B. leucithaltige Glaskeramiken, und nichtsilicatische Keramiken (Oxidkeramiken), wie etwa yttriumstabilisiertes Zirkonoxid, Aluminiumoxid und glasinfiltriertes Aluminiumoxid.

Die erfindungsgemäßen Haftvermittlerzusammensetzungen werden zur Herstellung eines Verbunds zwischen einem radikalisch härtenden Dentalmaterial und einem anderen dentalen Restaurationsmaterialien bevorzugt wie folgt angewandt:

Die Oberfläche des dentalen Restaurationsmaterials wird vor Auftragung der Haftvermittlerzusammensetzung konditioniert, wobei die Art der Konditionierung von der Art des Restaurationsmaterials abhängt. So werden silicatische Keramiken, einschließlich Glaskeramik, typischerweise chemisch geätzt, vorzugsweise mit Flusssäure, während metallische Materialien und nichtsilicatische Keramiken (Oxidkeramiken) typischerweise sandgestrahlt werden, etwa unter Verwendung von Korund. Der beim Sandstrahlen angewandte Druck beträgt vorzugsweise maximal 10⁵ Pa (1 bar).

Dann wird die konditionierte Oberfläche des Restaurationsmaterials gründlich mit Wasser gespült und anschließend getrocknet, etwa durch Verblasen des Wassers mit trockener, ölfreier Luft.

Auf die so konditionierte und trockene Oberfläche des Restaurationsmaterials wird die erfindungsgemäße Haftvermittlerzusammensetzung aufgetragen, typischerweise in einer dünnen Schicht und vorzugsweise mit einem feinen Pinsel (z. B. der Marke Microbrush®), und dann kurz einwirken gelassen. Anschließend wird die Haftvermittlerschicht getrocknet, etwa durch Verblasen mit trockener, ölfreier Luft.

Im letzten Schritt wird schließlich das radikalisch härtenden Dentalmaterial aufgebracht, um den gewünschten Verbund zwischen radikalisch härtenden Dentalmaterial und anderem dentalen Restaurationsmateriali herzustellen. Vor dem Aufbringen des radikalisch härtenden Dentalmaterials sollte die getrocknete Haftvermittlerschicht vorzugsweise nicht mit Flüssigkeiten, wie etwa Wasser, Speichel oder Blut, in Kontakt gebracht werden.

Die Erfindung wird im Folgenden anhand von Beispielen näher erläutert.

### Ausführungsbeispiele

### Beispiel 1

### Herstellung von Methacrylsäure-(3-trimethoxysilylpropyl)amid

Unter Argon wurden 77,4 g 3-(Amino)propyltrimethoxysilan, 43,7 g Triethylamin und 25 mg Di-tert.-butyl-*p*-kresol in 500 ml Dichlormethan gelöst. Bei -5 °C wurden 45,1 g Methacryloylchlorid langsam innerhalb 1 h zugetropft, anschließend wurde noch 1 h bei 0 °C nachgerührt. Das ausgefallene Hydrochlorid wurde abfiltriert und mit Dichlormethan nachgewaschen. Die flüchtigen Bestandteile wurden bei 40 °C unter vermindertem Druck am Rotationsverdampfer entfernt. Zurück blieb eine gelbe Flüssigkeit mit abgeschiedenem Feststoff (Hydrochlorid). Die Fällung des Hydrochlorids wurde durch Zugabe von 150 ml Diethylether vervollständigt, der Niederschlag wurde abfiltriert und das Filtrat unter Einleitung trockener Luft bei 40 °C am Rotationsverdampfer eingeengt. Die bräunliche Flüssigkeit wurde bei 4 Pa (4 x 10⁻² mbar) von restlichen flüchtigen Bestandteilen befreit und das Rohprodukt (104,2 g) wurde bei einem Druck von 0,06 Pa (6 x 10⁻⁴ mbar) destilliert. Das Produkt hatte dabei einen Siedepunkt von 123-125 °C. Es wurden 76,4 g Produkt als gelbliche, klare Flüssigkeit erhalten.

### Beispiel 2

### Herstellung von Liponsäure-2-ethoxycarbonylallylester LSEAE

Liponsäure-2-ethoxycarbonylallylester LSEAE wurde wie folgt in zwei Stufen hergestellt.

In der ersten Stufe wurde Ethyl-2-hydroxymethylacrylat durch Hydroxymethylierung von Ethylacrylat mit Paraformaldehyd in Gegenwart von Diazabicyclo[2.2.2]octan (DABCO) hergestellt.

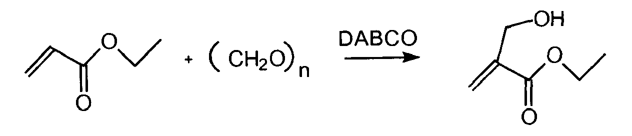

In einem zweiten Schritt lieferte die Reaktion von α-Liponsäure mit Ethyl-2-hydroxymethylacrylat in Gegenwart von 4-N,N-Dimethylaminopyridin und N-(3-Dimethylaminopropyl)-N'-ethylcarbodiimid-Hydrochlorid den Liponsäure-2-ethoxycarbonylallylester (LSEAE).

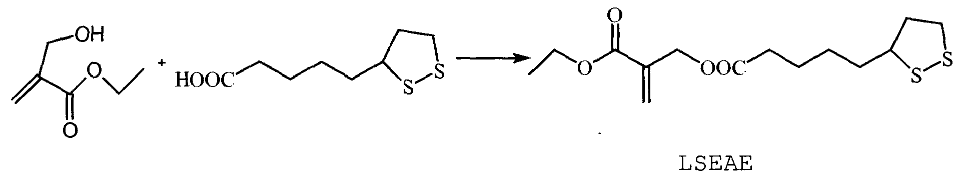

### Stufe 1: Herstellung von 2-Hydroxymethylacrylsäureethylester

Die Herstellung von 2-Hydroxymethylacrylsäureethylester erfolgte nach der Vorschrift von L. J. Mathias, H. Kusefoglu, Macromolec. 20 (1987) 2039-2041.

Eine Lösung von 440 g (4,4 mol) Ethylacrylat, 152 g (5,1 mol) Paraformaldehyd, 48 g (0,43 mol) Diazabicyclo[2.2.2]octan (DABCO) und 1 g Hydrochinonmonomethylether in 750 ml Dimethylsulfoxid wurde 16 h bei 65 °C gerührt. Nach dem Zugeben von 880 ml Wasser wurde mit 550 ml Diethylether extrahiert. Die wässrige Phase wurde noch 4 mal mit je 250 ml Ether extrahiert. Dann wurden die vereinigten Etherphasen mit 200 ml 0,5 N HCl und 2x mit 200 ml ges. Kochsalzlösung gewaschen und anschließend über wasserfreiem Natriumsulfat getrocknet. Nach Entfernen des Trockenmittels durch Filtration wurde das Lösungsmittel am Rotationsverdampfer unter Lufteinleiten abgezogen und der Rückstand wurde im Feinvakuum destilliert. Ausbeute: 168 g (34%) einer farblosen Flüssigkeit.

### Stufe 2: Herstellung von Liponsäure-2-ethoxycarbonylallylester (LSEAE)

Es wurden 2,60 g (20 mmol) 2-Hydroxymethylacrylsäureethylester, 4,13 g (20 mmol) α-Liponsäure, 122 mg (1,0 mmol) 4-N,N-Dimethylaminopyridin und 5 mg Butylhydroxytoluol (BHT) in 40 ml Methylenchlorid, das über ein Molekularsieb 4 Å getrocknet worden war, gelöst. Dann wurde eine Lösung von 4,54 g (22,0 mmol) N,N'-Dicyclohexylcarbodiimid in 10 ml Methylenchlorid zugetropft und es entstand ein weißer Niederschlag. Das Reaktionsgemisch wurde dann noch 6 h gerührt, bevor der Niederschlag aus der Reaktionsmischung mithilfe einer Nutsche abfiltriert wurde. Der Filterkuchen wurde noch 3x mit je 10 ml Methylenchlorid gewaschen und die vereinigten Filtrate wurde 3x mit je 50 ml gesättigter Natriumchlorid-Lösung gewaschen. Anschließend wurde die organische Phase über wasserfreiem Natriumsulfat getrocknet und das Trockenmittel abfiltriert. Das Lösungsmittel wurde am Rotationsverdampfer unter Einleiten eines schwachen trockenen Luftstroms abgezogen und der Rückstand wurde durch Säulenchromatographie mit n-Hexan / Ethylacetat gereinigt. Ausbeute: 4,58 g (72 %) eines gelben Öls.

### Beispiel 3

### Verwendete Alkoxysilan-Monomere (i)

### 3-Methacryloyloxypropyltrimethoxysilan (kommerziell erhältlich von Fluka und Evonik Degussa GmbH),

### Methacrylsäure-(3-trimethoxysilylpropyl)amid (Beispiel 1)

### Verwendete Phosphorsäureester-Monomere (ii)

1-Methacryloyloxydecan-10-phosphat wurde wie in US 4,612,384 beschrieben in einer Ausbeute von 75% und einer Reinheit von 95% (bestimmt durch HPLC) hergestellt.

1-Methacryloylaminodecan-10-phosphat wurde wie in EP 1 674 066 beschrieben in einer Ausbeute von 69% und einer Reinheit von 93% (bestimmt durch HPLC) hergestellt.

### Verwendete schwefelhaltige Monomere (iii)

### 2,2-Bisacryloylamino-diethyldisulfid (kommerziell erhältlich von Fluka)

### Liponsäure-2-ethoxycarbonylallylester (LSEAE; Beispiel 2)

### Herstellung der Haftvermittler (Primer)

### Haftvermittlerzusammensetzung A

1,0 Gew.-% 1-Methacryloyloxydecan-10-phosphat, 2,0 Gew.-% 3-Methacryloyloxypropyltrimethoxysilan und 1,0 Gew.-% Liponsäure-2-ethoxycarbonylallylester gelöst in 96 Gew.-% Ethanol puriss.

### Haftvermittlerzusammensetzung B

1,0 Gew.-% 1-Methacryloylaminodecan-10-phosphat, 2,0 Gew.-% Methacrylsäure-(3-trimethoxysilylpropyl)amid und 1,0 Gew.-% Liponsäure-2-ethoxycarbonylallylester gelöst in 96 Gew.-% Ethanol puriss.

### Haftvermittlerzusammensetzung C

1,0 Gew.-% 1-Methacryloyloxydecan-10-phosphat, 2,0 Gew.-% 3-Methacryloyloxypropyltrimethoxysilan und 1,0 Gew.-% 2,2-Bisacryloylamino-diethyldisulfid gelöst in 96 Gew.-% Ethanol puriss.

### Haftvermittlerzusammensetzung D

1,0 Gew.-% 1-Methacryloyloxydecan-10-phosphat, 3,0 Gew.-% 3-Methacryloyloxypropyltrimethoxysilan, 1,0 Gew.-% Liponsäure-2-ethoxycarbonylallylester gelöst in 93,5 Gew.-% Ethanol puriss. Dazu wurden 1,5 Gew.-% Aerosil® 200 (hydrophile pyrogene Kieselsäure mit einer BET-Oberfläche von etwa 200 m²/g und einer mittleren Größe der Primärteilchen von 12 nm, erhältlich von Evonik Degussa GmbH) gegeben und die Haftvermittlerzusammensetzung wurde in einer Ultraschalldurchflusszelle homogenisiert.

### Beispiel 4

### Bestimmung der Haftwerte auf unterschiedlichen dentalen Restaurationsmaterialien

Zur Bestimmung der Haftwerte wurde eine Abzugsanordnung angewendet wie sie in der Literatur beschrieben ist (M. Kern, V.P. Thompson, J. Prost. Dent. 1995 73(3): 240-249; M. Kern, V.P. Thompson, "Eine einfache Versuchsanordnung zur universellen Prüfung des Klebeverbundes im axialen Zugtest", Dtsch Zahnärztl Z 1993, 48: 769-772). Die erzielten Ergebnisse sind in der Tabelle 1 aufgeführt.

Die Herstellung der Prüfkörper erfolgte in enger Anlehnung an das in Dtsch Zahnärztl Z 1993, 48: 769-772 beschriebene Protokoll. Die würfelförmigen Prüfkörper wurden unter Wasserkühlung mit SiC-Schleifpapier der Korngrößen P120 - P400 - P1000 plan geschliffen.

Die einzelnen Substratoberflächen wurden gemäß den entsprechenden Herstelleranweisungen wie folgt konditioniert. So wurden silicatische Oberflächen mit Flusssäure geätzt und oxidische oder metallische Oberflächen mit entsprechender Körnung und Druck sandgestrahlt.
- E.max CAD wurde für 20 s mit dem Flusssäure-Gel "Ceramic Etch" kontaktiert und anschließend sorgfältig mit destilliertem Wasser abgespült.
- ZirCAD, Al-Cube, Tritan , Wiron 99 und d.Sign 91 wurden mit 50 µm Aluoxid-Strahlmittel (Korox 50) bei 2,5 x 10⁵ Pa (2,5 bar) Druck aus ca.1-2 cm Abstand für 15 s angeraut.

Alle Proben wurden anschließend für 10 min in iso-Propanol in einem Ultraschallbad gereinigt. Nach Entnahme aus dem iso-Propanol wurden die Proben mit Druckluft trockengeblasen und staubgeschützt bis zum Einsatz aufbewahrt.

Die Proben wurden mit einem mit Primer getränktem Microbrush®-Pinsel einmalig satt mit dem entsprechenden Haftvermittler bestrichen und die Flüssigkeit wurde 60 s einwirken lassen. Anschließend wurde überstehende Flüssigkeit mit Druckluft abgeblasen. Auf die geprimte Oberfläche wurde, wie in Dtsch Zahnärztl Z 1993, 48: 769-772 beschrieben, eine mit lichtpolymerisiertem Multicore Flow Komposit (Ivoclar Vivadent AG, Schaan, Liechtenstein) gefüllte Plexiglashülse aufgebracht. Dazu wurde die Hülse am zu haftenden Ende mit einem Tropfen Multilink Automix Zement (Ivoclar Vivadent AG, Schaan, Liechtenstein) belegt und mittels einer Anpressapparatur auf den Keramikprobekörper gebracht. Dann wurde der Zement durch 2 x 20 s Bestrahlung mit einer BluePhase Polymersationslampe ausgehärtet und die Proben wurden für 24 h bei 37 °C in Wasser gelagert. Danach wurde die Zughaftung mit der in der genannten Literatur beschriebenen Apparatur und einer Universal-Test-Maschine Z010 (Zwick-Roell, Ulm, Germany) bestimmt.

Zur Simulierung einer Dauerbelastung wurden die Prüfkörper auch einer Thermowechselbelastung unterzogen. Hierzu wurden die Prüfkörper vor der Zughaftfestigkeitsessung 5000 mal von 5°C kaltem Wasser in 55 °C warmes Wasser und zurück übertragen und jeweils für 60 s in dem Wasser belassen.

Die erfindungsgemäßen Haftvermittlerzusammensetzungen A, B, C und D wurden mit den folgenden kommerziell erhältlichen Keramik- bzw. Metallprimern verglichen:
- CP =: Clearfil Ceramic Primer von Kuraray Medical Inc., Okayama, Japan (auf Basis von 3-Methacryloyloxypropyltrimethoxysilan und 10-Methyacryloyloxydecyl-dihydrogenphosphat)
- AP =: Alloy Primer von Kuraray Medical Inc., Okayama, Japan (auf Basis von 6-(4-Vinylbenzyl-N-propyl)amino-1,3,5-triazin-2,4-dithion und 10-Methyacryloyloxydecyldihydrogenphosphat)
- MZP =: Metal Zirconia Primer von Ivoclar Vivadent AG, Schaan, Liechtenstein (auf Basis von Phosphonsäuremethacrylat)
- MS =: Monobond-S Primer von Ivoclar Vivadent AG, Schaan, Liechtenstein (auf Basis von 3-Methacryloyloxypropyltrimethoxysilan)

**Tabelle 1**

| **Haftvermittlerzusammensetzung** | **Anfangszughaftfestigkeit** **[MPa]** | **Zughaftfestigkeit nach Thermowechselbelastung** **[MPa]** |
|---|---|---|
| Substratoberfläche: e.max CAD (Leucit verstärkte Glaskeramik von Ivoclar Vivadent AG) | | |
| A | 54,9 ± 5,5 | 55,1 ± 10,1 |
| B | 52,3 ± 8,1 | 49,8 ± 6,9 |
| C | 49,1 ± 9,4 | 47,2 ± 7,49 |
| D | 50,8 ± 7,9 | 51,9 ± 11,0 |

| Substratoberfläche: ZirCAD (Zirkonoxidkeramik von Ivoclar Vivadent AG) | | |
|---|---|---|
| A | 44,8 ± 7,2 | 39,8 ± 8,2 |
| D | 39,5 ± 10,4 | 40,30 ± 5,9 |
| CP* | 40,1 ± 4,5 | 35,3 ± 11,6 |
| AP* | 31,1 ± 8,9 | 26,9 ± 10,1 |
| MZP* | 27,8 ± 4,5 | 25,7 ± 8,1 |
| MS* | 9,3 ± 3,4 | - |

| Substratoberfläche: Al-Cube (Aluminiumoxidkeramik von VITA Zahnfabrik, Bad Säckingen) | | |
|---|---|---|
| A | 39,4 ± 11,3 | 20,9 ± 14,4 |
| B | 38,8 ± 9,9 | 22,3 ± 8,2 |
| CP* | 22,2 ± 4 | 15 ± 12,1 |
| Substratoberfläche: Tritan (99,5% Titan von Dentaurum, Ispringen) | | |
| A | 39,5 ± 5,6 | 21,7 ± 16,8 |
| AP* | 17,5 ± 8,5 | 11,3 ± 6,6 |

| Substratoberfläche: Wiron 99 (Ni/Cr/Mo-Legierung von BEGO, Bremen) | | |
|---|---|---|
| A | 40,3 ± 8,6 | 27,6 ± 4,9 |
| B | 34,3 ± 9,1 | 24,9 ± 10,1 |
| AP* | 28,5 ± 12,8 | 25 ± 12,2 |
| MZP* | 23,1 ± 4,8 | 14,9 ± 9,49 |

| Substratoberfläche: d.Sign 91 (Au/Pd/Ga-Legierung von Ivoclar Vivadent AG) | | |
|---|---|---|
| A | 29,7 ± 5,3 | 20,1 ± 8,2 |
| AP* | 15,7 ± 6,6 | 13,2 ± 6,8 |

| | | |
|---|---|---|
| * Vergleichsbeispiel | | |

Wie die Haftwerte in der Tabelle 1 zeigen, zeichnen sich die erfindungsgemäßen Primer A, B, C und D durch einen sehr guten Verbund zu allen getesteten Substratoberflächen aus und verfügen über eine sehr gute Thermolastbeständigkeit. Sie verbinden die guten Haftwerte der kommerziellen, aber auf bestimmte Oberflächentypen beschränkten Formulierungen CP, AP, MZP und MS mit einer leichteren Handhabung. Verglichen mit den spezialisierten Primern erlauben A, B, C und D damit im klinischen Alltag eine wesentlich Vereinfachung der Arbeitsabläufe ohne Einbußen bei den erzielten Eigenschaften hinnehmen zu müssen.

## Patentansprüche

1. Haftvermittlerzusammensetzung enthaltend:
(i) mindestens ein Alkoxysilan-Monomer der allgemeinen Formel
R¹ₙsi(OR²)₄₋ₙ (I),
worin
R¹ für einen Rest steht, der mindestens eine polymerisierbare Gruppe aufweist,
R² für einen C₁- bis C₈-Alkylrest steht und
n 1,2 oder 3 ist,
wobei die Reste R¹ und R² jeweils gleich oder unterschiedlich sein können;
(ii) mindestens ein Phosphorsäureester-Monomer der allgemeinen Formel
O=P(OR³)ₘ(OR⁴)₃₋ₘ (II),
worin
R³ für einen Rest steht, der mindestens eine polymerisierbare Gruppe aufweist,
R⁴ für einen Rest ausgewählt aus H, Silyl und C₁- bis C₁₆-Alkyl steht und
m 1 oder 2 ist,
wobei die Reste R³ und R⁴ jeweils gleich oder unterschiedlich sein können;
(iii) mindestens ein schwefelhaltiges Monomer der allgemeinen Formel
R⁵-R⁶-Sₓ-R⁶-R⁵ (IIIa)
oder
R⁵-R⁶-Sₓ-R⁷ (IIIb),
worin
x eine ganze Zahl von 1 bis 8, ist,
R⁵ für einen Rest steht, der mindestens eine polymerisierbare Gruppe aufweist, und die beiden R⁵ in Formel (IIIa) gleich oder unterschiedlich sein können,
R⁶ für einen C₁- bis C₃₀-Alkylenrest steht, und die beiden R⁶ in Formel (IIIa) gleich oder unterschiedlich sein können,
R⁷ für H oder einen C₁- bis C₂₆-Alkylrest steht,
wobei die beiden R⁶ in Formel (IIIa) bzw. R⁶ und R⁷ in Formel (IIIb) miteinander verbunden sein können, um mit Sₓ einen Heterocyclus zu bilden, und
(iv) organisches Lösungsmittel.

2. Haftvermittlerzusammensetzung gemäß Anspruch 1 enthaltend (i) ein Alkoxysilan-Monomer der allgemeinen Formel (I), worin R¹ für einen Rest steht, der eine ethylenisch ungesättigte Doppelbindung aufweist.

3. Haftvermittlerzusammensetzung gemäß Anspruch 2 enthaltend (i) ein Alkoxysilan-Monomer der allgemeinen Formel (I), worin R¹ für einen Rest steht, der eine Gruppe ausgewählt aus (Meth)acryloyloxy, (Meth)acryloylamino, Vinyl, Allyl und Styryl aufweist.

4. Haftvermittlerzusammensetzung gemäß Anspruch 3 enthaltend (i) ein Alkoxysilan-Monomer der allgemeinen Formel (I), worin R¹ für einen Rest steht, der ausgewählt ist aus (Meth)acryloyloxyalkyl, (Meth)acryloylaminoalkyl, Vinyl und Allyl.

5. Haftvermittlerzusammensetzung gemäß einem der vorstehenden Ansprüche enthaltend (ii) ein Phosphorsäureester-Monomer der allgemeinen Formel (II), worin R³ für einen Rest steht, der eine ethylenisch ungesättigte Doppelbindung aufweist.

6. Haftvermittlerzusammensetzung gemäß Anspruch 5 enthaltend (ii) ein Phosphorsäureester-Monomer der allgemeinen Formel (II), worin R³ für einen Rest steht, der eine Gruppe ausgewählt aus (Meth)acryloyloxy, (Meth)acryloylamino, Vinyl, Allyl und Styryl aufweist.

7. Haftvermittlerzusammensetzung gemäß Anspruch 6 enthaltend (ii) ein Phosphorsäureester-Monomer der allgemeinen Formel (II), worin R³ für einen Rest steht, der ausgewählt ist aus (Meth)acryloyloxyalkyl, (Meth)acryloylaminoalkyl, Vinyl, Allyl und Styryl.

8. Haftvermittlerzusammensetzung gemäß einem der vorstehenden Ansprüche enthaltend (iii) ein schwefelhaltiges Monomer der allgemeinen Formel (IIIa) oder (IIIb), worin R⁵ für einen Rest steht, der eine ethylenisch ungesättigte Doppelbindung aufweist.

9. Haftvermittlerzusammensetzung gemäß Anspruch 8 enthaltend (iii) ein schwefelhaltiges Monomer der allgemeinen Formel (IIIa) oder (IIIb), worin R⁵ für einen Rest steht, der eine Gruppe ausgewählt aus (Meth)acryloyloxy, (Meth)acryloylamino, Vinyl, Allyl und Styryl aufweist.

10. Haftvermittlerzusammensetzung gemäß Anspruch 9 enthaltend (iii) ein schwefelhaltiges Monomer der allgemeinen Formel (IIIa) oder (IIIb), worin R⁵ für einen Rest steht, der ausgewählt ist aus (Meth)acryloyloxy, (Meth)acryloylamino, einer unsubstituierten oder substituierten Vinylgruppe, einer unsubstituierten oder substituierten Allylgruppe, einer unsubstituierten oder substituierten Styrylgruppe, einer unsubstituierten oder substituierten Vinylethergruppe, einer unsubstituierten oder substituierten Vinylestergruppe, einer unsubstituierten oder substituierten Allylethergruppe, einer unsubstituierten oder substituierten Allylestergruppe und Vinylcyclopropyl.

11. Haftvermittlerzusammensetzung gemäß einem der vorstehenden Ansprüche enthaltend (iii) ein schwefelhaltiges Monomer der allgemeinen Formel (IIIa) oder (IIIb), worin die beiden R⁶ in Formel (IIIa) bzw. R⁶ und R⁷ in Formel (IIIb) mit Sₓ einen Dithiolanring bilden.

12. Haftvermittlerzusammensetzung gemäß einem der vorstehenden Ansprüche, enthaltend
(a) 0,05 bis 25,0 Gew.-% Alkoxysilan-Monomer(e) (i);
(b) 0,05 bis 25,0 Gew.-% Phosphorsäureester-Monomer(e) (ii);
(c) 0,05 bis 25,0 Gew.-% Monomer(e) (iii);
(d) 25 bis 98,5 Gew.-% organisches Lösungsmittel (iv);
(e) 0 bis 10 Gew.-% Füllstoff (v),
(f) 0,001 bis 3 Gew.-% Polymerisationsinitiator (vi),
jeweils bezogen auf das Gesamtgewicht der Zusammensetzung.

13. Haftvermittlerzusammensetzung gemäß einem der vorstehenden Ansprüche zur Verwendung in der Zahnheilkunde.

14. Haftvermittlerzusammensetzung gemäß Anspruch 13 zur adhäsiven Verbindung von metallischen oder keramischen Dentalmaterialien mit radikalisch härtenden Dentalmaterialien.

15. Verwendung der Haftvermittlerzusammensetzung gemäß einem der Ansprüche 1 bis 12 in der Zahntechnik.

16. Verwendung nach Anspruch 15, wobei die Haftvermittlerzusammensetzung zur adhäsiven Verbindung von metallischen oder keramischen Dentalmaterialien mit radikalisch härtenden Dentalmaterialien verwendet wird.

## Claims

1. Adhesion-promoter composition containing:
(i) at least one alkoxysilane monomer of the general formula
R¹ₙsi(OR²)₄₋ₙ (I),
in which
R¹ stands for a group that has at least one polymerisable group,
R² stands for a C₁ to C₈ alkyl group and
n is 1, 2 or 3,
wherein the groups R¹ and R² can each be the same or different;
(ii) at least one phosphoric acid ester monomer of the general formula
O=P(OR³)ₘ(OR⁴)₃₋ₘ (II),
in which
R³ stands for a group that has at least one polymerisable group,
R⁴ stands for a group selected from H, silyl and C₁ to C₁₈ alkyl and
m is 1 or 2,
wherein the groups R³ and R⁴ can each be the same or different;
(iii) at least one sulfur-containing monomer of the general formula
R⁵-R⁶-Sₓ-R⁶-R⁵ (IIIa)
or
R⁵-R⁸-Sₓ-R⁷ (IIIb),
in which
x is an integer from 1 to 8,
R⁵ stands for a group that has at least one polymerisable group and both the R⁵ in formula (IIIa) can be the same or different,
R⁶ stands for a C₁ to C₃₀ alkylene group and both the R⁶ in formula (IIIa) can be the same or different,
R⁷ stands for H or a C₁ to C₂₆ alkyl group,
wherein the two R⁶ in formula (IIIa) or R⁶ and R⁷ in formula (IIIb) can be linked to each other to form a heterocycle with Sₓ, and
(iv) organic solvent.

2. Adhesion-promoter composition according to claim 1 comprising (l) an alkoxysilane monomer of the general formula (l), in which R¹ stands for a group that has an ethylenically unsaturated double bond.

3. Adhesion-promoter composition according to claim 2 comprising (l) an alkoxysilane monomer of the general formula (l), in which R¹ stands for a group that has a group selected from (meth)acryloyloxy, (meth)acryloylamino, vinyl, allyl and styryl.

4. Adhesion-promoter composition according to claim 3 comprising (i) an alkoxysilane monomer of the general formula (I), in which R¹ stands for a group that is selected from (meth)acryloyloxyalkyl, (meth)acryloylaminoalkyl, vinyl and allyl.

5. Adhesion-promoter composition according to one of the preceding claims comprising (ii) a phosphoric acid ester monomer of the general formula (II), in which R³ stands for a group that has an ethylenically unsaturated double bond.

6. Adhesion-promoter composition according to claim 5 comprising (ii) a phosphoric acid ester monomer of the general formula (II), In which R³ stands for a group that has a group selected from (meth)acryloyloxy, (meth)acryloylamino, vinyl, allyl and styryl.

7. Adhesion-promoter composition according to claim 6 comprising (ii) a phosphoric acid ester monomer of the general formula (II), in which R³ stands for a group that is selected from (meth)acryloyloxyalkyl, (meth)acryloylaminoalkyl, vinyl; allyl and styryl.

8. Adhesion-promoter composition according to one of the preceding claims comprising (iii) a sulfur-containing monomer of the general formula (IIIa) or (IIIb), in which R⁵ stands for a group that has an ethylenically unsaturated double bond.

9. Adhesion-promoter composition according to claim 8 comprising **(III)** a sulfur-containing monomer of the general formula (IIIa) or (IIIb), in which R⁵ stands for a group that has a group selected from (meth)acryloyloxy, (meth)acryloylamino, vinyl, allyl and styryl.

10. Adhesion-promoter composition according to claim 9 comprising (iii) a sulfur-containing monomer of the general formula (IIIa) or (IIIb), in which R⁵ stands for a group that is selected from (meth)acryloyloxy, (meth)acryloylamino, an unsubstituted or substituted vinyl group, an unsubstituted or substituted allyl group, an unsubstituted or substituted styryl group, an unsubstituted or substituted vinyl ether group, an unsubstituted or substituted vinyl ester group, an unsubstituted or substituted allyl ether group, an unsubstituted or substituted allyl ester group and vinylcyclopropyl.

11. Adhesion-promoter composition according to one of the preceding claims comprising (iii) a sulfur-containing monomer of the general formula (IIIa) or (IIIb), in which both the R⁶ in formula (IIIa) or R⁶ and R⁷ in formula (IIIb) form a dithiolane ring with Sₓ.

12. Adhesion-promoter composition according to one of the preceding claims, comprising
(a) 0.05 to 25.0 % by weight alkoxysilane monomer(s) (I);
(b) 0.05 to 25.0 % by weight phosphoric acid ester monomer(s) (ii);
(c) 0.05 to 25.0 % by weight monomer(s) (iii);
(d) 25 to 98.5 % by weight organic solvent (iv);
(e) 0 to 10 % by weight filler (v),
(f) 0.001 to 3 % by weight polymerisation initiator (vi);
in each case based on the total weight of the composition.

13. Adhesion-promoter composition according to one of the preceding claims for use in dentistry.

14. Adhesion-promoter composition according to claim 13 for the adhesive joining of metallic or ceramic dental materials with radically curing dental materials.

15. Use of the adhesion-promoter composition according to one of claims 1 to 12 in dental engineering.

16. Use according to claim 15, wherein the adhesion-promoter composition is used for the adhesive joining of metallic or ceramic dental materials with radically curing dental materials.

## Revendications

1. Composition d'adhésif contenant :
i) au moins un monomère de type alcoxy-silane, de formule générale
R¹ₙSi(OR²)₄₋ₙ (I)
dans laquelle
- R¹ représente un fragment qui comporte au moins un groupe polymérisable ;
- R² représente un groupe alkyle en C₁-C₈,
- et l'indice n vaut 1, 2 ou 3,
étant entendu que les entités représentées par R¹ ou R² peuvent être, dans chaque cas, identiques ou différentes ;
ii) au moins un monomère de type ester d'acide phosphorique, de formule générale
O=P(OR³)ₘ(OR⁴)₃₋ₘ (II)
dans laquelle
- R³ représente un fragment qui comporte au moins un groupe polymérisable ;
- R⁴ représente une entité choisie parmi un atome d'hydrogène, un groupe silyle et un groupe alkyle en C₁-C₁₆,
- et l'indice m vaut 1 ou 2,
étant entendu que les entités représentées par R³ ou R⁴ peuvent être, dans chaque cas, identiques ou différentes ;
iii) au moins un monomère soufré, de formule générale
R⁵-R⁶-Sₓ-R⁶-R⁵ (IIIa)
ou
R⁵-R⁶-Sₓ-R⁷ (IIIb)
dans lesquelles formules
- l'indice x est un nombre entier valant de 1 à 8,
- R⁵ représente un fragment qui comporte au moins un groupe polymérisable, étant entendu que les deux fragments représentés par R⁵ dans la formule (IIIa) peuvent être identiques ou différents ;
- R⁶ représente un groupe alcanediyle en C₁-C₃₀, étant entendu que les deux fragments représentés par R⁶ dans la formule (IIIa) peuvent être identiques ou différents ;
- et R⁷ représente un atome d'hydrogène ou un groupe alkyle en C₁-C₂₆,
étant entendu que les deux groupes représentés par R⁶ dans la formule (IIIa), ou les groupes représentés par R⁶ et R⁷ dans la formule (IIIb), peuvent être raccordés l'un à l'autre pour former un hétérocycle avec les x atomes de soufre ;
iv) et un solvant organique.

2. Composition d'adhésif conforme à la revendication 1, contenant un monomère alcoxy-silane (i) de formule générale (I) dans laquelle R¹ représente un fragment insaturé comportant une double liaison éthylénique.

3. Composition d'adhésif conforme à la revendication 2, contenant un monomère alcoxy-silane (i) de formule générale (I) dans laquelle R¹ représente un fragment qui comporte un groupe choisi parmi les groupes acryloyl-oxy, méthacryloyl-oxy, acryloyl-amino, méthacryloyl-amino, vinyle, allyle et styryle.

4. Composition d'adhésif conforme à la revendication 3, contenant un monomère alcoxy-silane (i) de formule générale (I) dans laquelle R¹ représente un fragment qui est choisi parmi les groupes acryloyl-oxy-alkyle, méthacryloyl-oxy-alkyle, acryloyl-amino-alkyle, méthacryloyl-amino-alkyle, vinyle et allyle.

5. Composition d'adhésif conforme à l'une des revendications précédentes, contenant un monomère ester d'acide phosphorique (ii) de formule générale (II) dans laquelle R¹ représente un fragment insaturé comportant une double liaison éthylénique.

6. Composition d'adhésif conforme à la revendication 5, contenant un monomère ester d'acide phosphorique (ii) de formule générale (II) dans laquelle R³ représente un fragment qui comporte un groupe choisi parmi les groupes acryloyl-oxy, méthacryloyl-oxy, acryloyl-amino, méthacryloyl-amino, vinyle, allyle et styryle.

7. Composition d'adhésif conforme à la revendication 6, contenant un monomère ester d'acide phosphorique (ii) de formule générale (II) dans laquelle R³ représente un fragment qui est choisi parmi les groupes acryloyl-oxy-alkyle, méthacryloyl-oxy-alkyle, acryloyl-amino-alkyle, méthacryloyl-amino-alkyle, vinyle, allyle et styryle.

8. Composition d'adhésif conforme à l'une des revendications précédentes, contenant un monomère soufré (iii) de formule générale (IIIa) ou (IIIb) dans laquelle R⁵ représente un fragment insaturé comportant une double liaison éthylénique.

9. Composition d'adhésif conforme à la revendication 8, contenant un monomère soufré (iii) de formule générale (IIIa) ou (IIIb) dans laquelle R⁵ représente un fragment qui comporte un groupe choisi parmi les groupes acryloyl-oxy, méthacryloyl-oxy, acryloyl-amino, méthacryloyl-amino, vinyle, allyle et styryle.

10. Composition d'adhésif conforme à la revendication 9, contenant un monomère soufré (iii) de formule générale (IIIa) ou (IIIb) dans laquelle R⁵ représente un fragment qui est choisi parmi les groupes acryloyl-oxy, méthacryloyl-oxy, acryloyl-amino, méthacryloyl-amino, vinyle avec ou sans substituant(s), allyle avec ou sans substituant(s), styryle avec ou sans substituant(s), vinyl-éther avec ou sans substituant(s), vinyl-ester avec ou sans substituant(s), allyl-éther avec ou sans substituant(s), allyl-ester avec ou sans substituant(s), et vinyl-cyclopropyle.

11. Composition d'adhésif conforme à l'une des revendications précédentes, contenant un monomère soufré (iii) de formule générale (IIIa) ou (IIIb) dans laquelle les deux groupes représentés par R⁶ dans la formule (IIIa), ou les groupes représentés par R⁶ et R⁷ dans la formule (IIIb), forment un cycle dithiolane avec les x atomes de soufre.

12. Composition d'adhésif conforme à l'une des revendications précédentes, contenant
a) de 0,05 à 25,0 % en poids de monomère(s) (i) de type alcoxy-silane,
b) de 0,05 à 25,0 % en poids de monomère(s) (ii) de type ester d'acide phosphorique,
c) de 0,05 à 25,0 % en poids de monomère(s) (iii),
d) de 25 à 98,5 % en poids de solvant organique (iv),
e) de 0 à 10 % en poids de charge (v),
f) de 0,001 à 3 % en poids d'amorceur de polymérisation (vi),
chacun de ces pourcentages étant en poids rapporté au poids total de la composition.

13. Composition d'adhésif conforme à l'une des revendications précédentes, pour utilisation en chirurgie dentaire.

14. Composition d'adhésif conforme à la revendication 13, pour liaison par adhésion de matériaux dentaires métalliques ou céramiques avec des matériaux dentaires durcissables par voie radicalaire.

15. Utilisation d'une composition d'adhésif conforme à l'une des revendications 1 à 12 en technique dentaire.

16. Utilisation conforme à la revendication 15, dans laquelle la composition d'adhésif est utilisée pour assurer la liaison par adhésion de matériaux dentaires métalliques ou céramiques avec des matériaux dentaires durcissables par voie radicalaire.
